# EUROPEAN PATENT APPLICATION

(11) **EP 2 508 501 A2**
(43) Date of publication of application: **10.10.2012**
(21) Application number: 12163285.5
(22) Date of filing: 05.04.2012
(51) Int. Cl.: C07C 51/02, C07C 51/41, C07C 51/44, C07C 51/46, C07C 55/10

(54) **Methods and systems of producing dicarboxylic acids**

(30) Priority: 05.04.2011 US 201161471930 P; 02.04.2012 US 201213437160
(71) Applicant: BioAmber International S.A.R.L., 1140 Luxembourg (LU)
(72) Inventor: Dunuwila, Dilum, Princeton, New Jersey 08540 (US); Cockrem, Michael, Madison, Wisconsin 53705 (US)
(74) Representative: Patentanwälte Ruff, Wilhelm, Beier, Dauster & Partner

(57) **Abstract**

A method of producing succinic acid (SA) includes providing fermentation derived diammonium succinate (DAS) containing solution, converting the DAS containing solution to a solution containing a half-acid, half-salt of succinic acid (MXS) by reactive evaporation, crystallizing MXS from the MXS containing solution by cooling and/or evaporative crystallization, converting MXS to SA by biopolar membrane electrodialysis, anion exchange, cation exchange, or a combination thereof, and crystallizing SA from SA the containing solution generated during conversion of the MXS to SA by cooling and/or evaporative crystallization.

## Description

### RELATED APPLICATION

This application claims the benefit of US Provisional Application No. 61/471,930, filed April 5, 2011, and US non provisional No. 13/437,160 filed April 2, 2012 the subject matter of which is each hereby incorporated by reference.

### TECHNICAL FIELD

This invention relates to processes and systems for producing dicarboxylic acids such as, for example, succinic acid (SA) from fermentation derived solutions containing salts of SA and adipic acid (AA) from fermentation derived solutions containing salts of AA.

### BACKGROUND

Certain carbonaceous products of sugar fermentation are seen as replacements for petroleum-derived materials for use as feed stocks for the manufacture of carbon-containing chemicals. Such products include SA and AA. It would accordingly be desirable to have processes and systems for producing substantially pure SA and AA from fermentation broths containing salts of SA and AA, respectively.

For example, SA can be produced by microorganisms using fermentable carbon sources such as sugars as starting materials. However, most commercially viable, succinate producing microorganisms substantially neutralize the fermentation broth to maintain an appropriate pH for maximum growth, conversion and productivity. Typically, the pH of the fermentation broth is maintained at or near a pH of 7 by introduction of NH₃ or NH₄⁺ into the broth, thereby converting SA to diammonium succinate (DAS). DAS is converted to SA to derive SA from the fermentation broth. Similar methods apply for conversion of diammonium adipate (DAA) to AA.

The pH of the fermentation broth can be maintained at a desired pH by introduction of sodium, potassium, or magnesium bases or mixtures thereof, including mixtures with ammonium bases. The addition of bases causes the SA or AA to convert to other salts of SA or AA. Other bases may include K⁺, Na⁺ and Mg⁺², for example. (In the case of magnesium succinate (MgS), for example, there can be some confusion between two different chemical species. One species is magnesium succinate. The other is magnesium bis(hydrogen succinate) (Mg(HS)₂). Hereinafter, both species will be referred to as MgS for the sake of simplicity.)

There are, however, difficulties and problems in economically producing such dicarboxylic acids.

### SUMMARY

The invention provides a number of methods of producing SA. Those methods include but are not limited to at least the following.

The invention provides fermentation derived DXS-containing solutions, where DXS comprises at least some DAS and, optionally, at least one of disodium succinate (DNaS) or dipotassium succinate (DKS), distilling/evaporating the DXS-containing solution to form an overhead that comprises water and ammonia and a liquid bottoms that comprises MXS, where MXS is at least one of monoammonium succinate (MAS), monosodium succinate (MNaS) or monopotassium succinate (MKS), and at least some DXS, crystallizing the MXS into a solid from the DXS-containing solution by cooling/evaporative/antisolvent crystallization, dissolving the MXS solid in water, converting the MXS to a SA containing solution by at least one of electrodialysis, anion exchange using a cationic resin, and cation exchange using an anionic resin, and crystallizing by cooling/evaporative crystallization the SA from the SA containing solution generated during conversion of the MXS to SA.

The invention also provides a method of producing SA including providing a fermentation derived DAS-containing solution comprising DAS and magnesium succinate (MgS), distilling/evaporating the DAS-containing solution to form an overhead that comprises water and ammonia, and a liquid bottoms that comprises MgS, crystallizing the MgS into a solid from the DAS-containing solution by cooling/evaporative/antisolvent crystallization, dissolving the MgS solid in water, converting the MgS to an SA-containing solution by at least one of anion exchange using a cationic resin, and cation exchange using an anionic resin, and crystallizing by cooling/evaporative crystallization the SA from the SA containing solution generated during conversion of the MgS to SA.

The invention further provides a method of producing SA including providing a fermentation derived MXS-containing solution, where MXS comprises at least one of MAS, MNaS or MKS, optionally, adding at least one of SA, NH₃, NH₄⁺, Na⁺, and K⁺ to the broth to preferably maintain the pH of the broth below 6, distilling/evaporating the MXS-containing solution to form an overhead that comprises water and, optionally, ammonia, and a liquid bottoms that comprises MXS, crystallizing the MXS into a solid from the MXS-containing solution by cooling/evaporative/antisolvent crystallization, dissolving the MXS solid in water, converting the MXS to an SA-containing solution by at least one of electrodialysis, anion exchange using a cationic resin, and cation exchange using an anionic resin, and crystallizing by cooling/evaporative crystallization the SA from the SA containing solution generated during conversion of the MXS to SA.

The invention yet provides a method of producing SA including providing a fermentation derived MgS-containing solution, adding at least one of SA, NH₃, NH₄⁺, and Mg⁺² to the broth to preferably maintain the pH of the broth below 6, distilling/evaporating the MgS-containing solution to form an overhead that comprises water and, optionally, ammonia, and a liquid bottoms that comprises MgS, crystallizing the MgS into a solid from the MgS-containing solution by cooling/evaporative/antisolvent crystallization, dissolving the MgS solid in water, converting the MgS to an SA-containing solution by at least one of anion exchange using a cationic resin, and cation exchange using an anionic resin, and crystallizing by cooling/evaporative crystallization the SA from the SA containing solution generated during conversion of the MgS to SA.

The invention also provides fermentation derived DXA-containing solutions, where DXA comprises at least some DAA and, optionally, at least one of disodium adipate (DNaA) or dipotassium adipate (DKA), distilling/evaporating the DXA-containing solution to form an overhead that comprises water and ammonia and a liquid bottoms that comprises MXA, where MXA is at least one of monoammonium adipate (MAA), monosodium adipate (MNaA) or monopotassium adipate (MKA), and at least some DXA, crystallizing the MXA into a solid from the DXA-containing solution by cooling/evaporative/antisolvent crystallization, dissolving the MXA solid in water, converting the MXA to a AA containing solution by at least one of electrodialysis, anion exchange using a cationic resin, and cation exchange using an anionic resin, and crystallizing by cooling/evaporative crystallization the AA from the AA containing solution generated during conversion of the MXA to AA.

The invention also provides a method of producing AA including providing a fermentation derived DAA-containing solution comprising DAA and magnesium adipate (MgA), distilling/evaporating the DAA-containing solution to form an overhead that comprises water and ammonia, and a liquid bottoms that comprises MgA and at least some DAA, crystallizing the MgA into a solid from the DAA-containing solution by cooling/evaporative/antisolvent crystallization, dissolving the MgA solid in water, converting the MgA to an AA containing solution by at least one of electrodialysis, anion exchange using a cationic resin, and cation exchange using an anionic resin, and crystallizing by cooling/evaporative crystallization the AA from the AA containing solution generated during conversion of the MgA to AA.

The invention further provides a method of producing AA including providing a fermentation derived MXA-containing solution, where MXA comprises at least one of MAA, MNaA or MKA, optionally, adding at least one of AA, NH₃, NH₄⁺, Na⁺, and K⁺ to the broth to preferably maintain the pH of the broth below 6, distilling/evaporating the MXA-containing solution to form an overhead that comprises water and, optionally, ammonia, and a liquid bottoms that comprises MXA, crystallizing the MXA into a solid from the MXA-containing solution by cooling/evaporative/antisolvent crystallization, dissolving the MXA solid in water, converting the MXA to an AA-containing solution by at least one of electrodialysis, anion exchange using a cationic resin, and cation exchange using an anionic resin, and crystallizing by cooling/evaporative crystallization the AA from the AA containing solution generated during conversion of the MXA to AA.

The invention yet provides a method of producing AA including providing a fermentation derived MgA-containing solution, adding at least one of AA, NH₃, NH₄⁺, and Mg⁺² to the broth to preferably maintain the pH of the broth below 6, distilling/evaporating the MgA-containing solution to form an overhead that comprises water and, optionally, ammonia, and a liquid bottoms that comprises MgA, crystallizing the MgA into a solid from the MgA-containing solution by cooling/evaporative/antisolvent crystallization, dissolving the MgA solid in water, converting the MgA to an AA-containing solution by at least one of electrodialysis, anion exchange using a cationic resin, and cation exchange using an anionic resin, and crystallizing by cooling/evaporative crystallization the AA from the AA containing solution generated during conversion of the MgA to AA.

### BRIEF DESCRIPTION OF THE DRAWINGS

Fig. 1 is a block flow diagram illustrating one of our previous methods for producing SA from a DAS-containing fermentation broth.

Fig. 2 is a block flow diagram illustrating a process for converting a DAS- containing fermentation broth to MAS and further converting the MAS to SA using electrodialysis, wherein ammonia is shown as a base used for neutralizing the fermentation.

Fig. 3 is a block flow diagram illustrating a process for converting a DAS- containing fermentation broth to MAS and further converting the MAS to SA by anion exchange using cationic resins, wherein ammonia is shown as a base used for neutralizing the fermentation.

Fig. 4 is a block flow diagram illustrating a process for converting a DAS- containing fermentation broth to MAS and further converting the MAS to SA by cation exchange using anionic resins, wherein ammonia is shown as the base used for neutralizing the fermentation.

### DETAILED DESCRIPTION

A first aspect of the invention relates to a method of producing fermentation derived DXS-containing solutions, where DXS comprises at least some DAS and, optionally, at least one of disodium succinate (DNaS) or dipotassium succinate (DKS), distilling/evaporating the DXS-containing solution to form an overhead that comprises water and ammonia and a liquid bottoms that comprises MXS, where MXS is at least one of monoammonium succinate (MAS), monosodium succinate (MNaS) or monopotassium succinate (MKS), and at least some DXS, crystallizing the MXS into a solid from the DXS-containing solution by cooling/evaporative/antisolvent crystallization, dissolving the MXS solid in water, converting the MXS to a SA containing solution by at least one of electrodialysis, anion exchange using a cationic resin, and cation exchange using an anionic resin, and crystallizing by cooling/evaporative crystallization the SA from the SA containing solution generated during conversion of the MXS to SA.

In a preferred embodiment, distilling the DXS-containing solution is carried out in the presence of an ammonia separating solvent which is at least one selected from the group consisting of diglyme, triglyme, tetraglyme, sulfoxides, amides, sulfones, polyethyleneglycol (PEG), gamma butyrolactone (GBL), butoxytriglycol, N-methylpyrolidone (NMP), ethers, and methyl ethyl ketone (MEK) or in the presence of a water azeotroping solvent which is at least one selected from the group consisting of toluene, xylene, methylcyclohexane, methyl isobutyl ketone, hexane, cyclohexane and heptane.

The method can further comprise removing water from the liquid bottoms to increase concentration of MXS in the liquid bottoms.

The MXS solid is preferably substantially free, in particular completely free, of DXS, succinamic acid, succinamide and succinimide.

In a second aspect, the invention refers to a method of producing SA including providing a fermentation derived DAS-containing solution comprising DAS and magnesium succinate (MgS), distilling/evaporating the DAS-containing solution to form an overhead that comprises water and ammonia, and a liquid bottoms that comprises MgS, crystallizing the MgS into a solid from the DAS-containing solution by cooling/evaporative/antisolvent crystallization, dissolving the MgS solid in water, converting the MgS to an SA-containing solution by at least one of anion exchange using a cationic resin, and cation exchange using an anionic resin, and crystallizing by cooling/evaporative crystallization the SA from the SA containing solution generated during conversion of the MgS to SA.

In a preferred embodiment, distilling the DAS-containing solution is carried out in the presence of an ammonia separating solvent which is at least one selected from the group consisting of diglyme, triglyme, tetraglyme, sulfoxides, amides, sulfones, polyethyleneglycol (PEG), gamma butyrolactone (GBL), butoxytriglycol, N-methylpyrolidone (NMP), ethers, and methyl ethyl ketone (MEK) or in the presence of a water azeotroping solvent which is at least one selected from the group consisting of toluene, xylene, methylcyclohexane, methyl isobutyl ketone, hexane, cyclohexane and heptane.

The method can further comprise removing water from the liquid bottoms to increase concentration of MgS in the liquid bottoms.

Preferably, the MgS is substantially free, in particular completely free, of DAS, succinamic acid, succinamide and succinimide.

In a third aspect the invention pertains to a method of producing SA including providing a fermentation derived MXS-containing solution, where MXS comprises at least one of MAS, MNaS or MKS, optionally, adding at least one of SA, NH₃, NH₄⁺, Na⁺, and K⁺ to the broth to preferably maintain the pH of the broth below 6, distilling/evaporating the MXS-containing solution to form an overhead that comprises water and, optionally, ammonia, and a liquid bottoms that comprises MXS, crystallizing the MXS into a solid from the MXS-containing solution by cooling/evaporative/antisolvent crystallization, dissolving the MXS solid in water, converting the MXS to an SA-containing solution by at least one of electrodialysis, anion exchange using a cationic resin, and cation exchange using an anionic resin, and crystallizing by cooling/evaporative crystallization the SA from the SA containing solution generated during conversion of the MXS to SA.

In a preferred embodiment, distilling the MXS-containing solution is carried out in the presence of an ammonia separating solvent which is at least one selected from the group consisting of diglyme, triglyme, tetraglyme, sulfoxides, amides, sulfones, polyethyleneglycol (PEG), gamma butyrolactone (GBL), butoxytriglycol, N-methylpyrolidone (NMP), ethers, and methyl ethyl ketone (MEK) or in the presence of a water azeotroping solvent which is at least one selected from the group consisting of toluene, xylene, methylcyclohexane, methyl isobutyl ketone, hexane, cyclohexane and heptane.

The method can further comprise removing water from the liquid bottoms to increase concentration of MXS in the liquid bottoms.

Preferably, the solid MXS is substantially free, in particular completely free, of succinamic acid, succinamide and succinimide.

In a fourth aspect the invention encompasses a method of producing SA including providing a fermentation derived MgS-containing solution, adding at least one of SA, NH₃, NH₄⁺, and Mg⁺² to the broth to preferably maintain the pH of the broth below 6, distilling/evaporating the MgS-containing solution to form an overhead that comprises water and, optionally, ammonia, and a liquid bottoms that comprises MgS, crystallizing the MgS into a solid from the MgS-containing solution by cooling/evaporative/antisolvent crystallization, dissolving the MgS solid in water, converting the MgS to an SA-containing solution by at least one of anion exchange using a cationic resin, and cation exchange using an anionic resin, and crystallizing by cooling/evaporative crystallization the SA from the SA containing solution generated during conversion of the MgS to SA.

In a preferred embodiment, distilling the MgS-containing solution is carried out in the presence of an ammonia separating solvent which is at least one selected from the group consisting of diglyme, triglyme, tetraglyme, sulfoxides, amides, sulfones, polyethyleneglycol (PEG), gamma butyrolactone (GBL), butoxytriglycol, N-methylpyrolidone (NMP), ethers, and methyl ethyl ketone (MEK) or in the presence of a water azeotroping solvent which is at least one selected from the group consisting of toluene, xylene, methylcyclohexane, methyl isobutyl ketone, hexane, cyclohexane and heptane.

The method can further comprise removing water from the liquid bottoms to increase concentration of MgS in the liquid bottoms.

Preferably, the MgS solid is substantially free, in particular completely free, of succinamic acid, succinamide and succinimide.

In a fifth aspect, the invention relates to a method of producing fermentation derived DXA-containing solutions, where DXA comprises at least some DAA and, optionally, at least one of disodium adipate (DNaA) or dipotassium adipate (DKA), distilling/evaporating the DXA-containing solution to form an overhead that comprises water and ammonia and a liquid bottoms that comprises MXA, where MXA is at least one of monoammonium adipate (MAA), monosodium adipate (MNaA) or monopotassium adipate (MKA), and at least some DXA, crystallizing the MXA into a solid from the DXA-containing solution by cooling/evaporative/antisolvent crystallization, dissolving the MXA solid in water, converting the MXA to a AA containing solution by at least one of electrodialysis, anion exchange using a cationic resin, and cation exchange using an anionic resin, and crystallizing by cooling/evaporative crystallization the AA from the AA containing solution generated during conversion of the MXA to AA.

In a preferred embodiment, distilling the DXA-containing solution is carried out in the presence of an ammonia separating solvent which is at least one selected from the group consisting of diglyme, triglyme, tetraglyme, sulfoxides, amides, sulfones, polyethyleneglycol (PEG), gamma butyrolactone (GBL), butoxytriglycol, N-methylpyrolidone (NMP), ethers, and methyl ethyl ketone (MEK) or in the presence of a water azeotroping solvent which is at least one selected from the group consisting of toluene, xylene, methylcyclohexane, methyl isobutyl ketone, hexane, cyclohexane and heptane.

The method can further comprise removing water from the liquid bottoms to increase concentration of MXA in the liquid bottoms.

Preferably, the MXA solid is substantially free, in particular completely free, of DXA, adipamic acid, adipamide and adipimide.

In a sixth aspect, the invention refers to a method of producing AA including providing a fermentation derived DAA-containing solution comprising DAA and magnesium adipate (MgA), distilling/evaporating the DAA-containing solution to form an overhead that comprises water and ammonia, and a liquid bottoms that comprises MgA and at least some DAA, crystallizing the MgA into a solid from the DAA-containing solution by cooling/evaporative/antisolvent crystallization, dissolving the MgA solid in water, converting the MgA to an AA containing solution by at least one of electrodialysis, anion exchange using a cationic resin, and cation exchange using an anionic resin, and crystallizing by cooling/evaporative crystallization the AA from the AA containing solution generated during conversion of the MgA to AA.

In a preferred embodiment, distilling the broth and/or the DAA-containing solution is carried out in the presence of an ammonia separating solvent which is at least one selected from the group consisting of diglyme, triglyme, tetraglyme, sulfoxides, amides, sulfones, polyethyleneglycol (PEG), gamma butyrolactone (GBL), butoxytriglycol, N-methylpyrolidone (NMP), ethers, and methyl ethyl ketone (MEK) or in the presence of a water azeotroping solvent which is at least one selected from the group consisting of toluene, xylene, methylcyclohexane, methyl isobutyl ketone, hexane, cyclohexane and heptane.

The method can further comprise removing water from the liquid bottoms to increase concentration of MgA in the liquid bottoms.

Preferably, the MgA solid is substantially free, in particular completely free, of DAA, adipamic acid, adipamide and adipimide.

In a seventh aspect, the invention pertains to a method of producing AA including providing a fermentation derived MXA-containing solution, where MXA comprises at least one of MAA, MNaA or MKA, optionally, adding at least one of AA, NH₃, NH₄⁺, Na⁺, and K⁺ to the broth to preferably maintain the pH of the broth below 6, distilling/evaporating the MXA-containing solution to form an overhead that comprises water and, optionally, ammonia, and a liquid bottoms that comprises MXA, crystallizing the MXA into a solid from the MXA-containing solution by cooling/evaporative/antisolvent crystallization, dissolving the MXA solid in water, converting the MXA to an AA-containing solution by at least one of electrodialysis, anion exchange using a cationic resin, and cation exchange using an anionic resin, and crystallizing by cooling/evaporative crystallization the AA from the AA containing solution generated during conversion of the MXA to AA.

In a preferred embodiment, distilling the MXA-containing solution is carried out in the presence of an ammonia separating solvent which is at least one selected from the group consisting of diglyme, triglyme, tetraglyme, sulfoxides, amides, sulfones, polyethyleneglycol (PEG), gamma butyrolactone (GBL), butoxytriglycol, N-methylpyrolidone (NMP), ethers, and methyl ethyl ketone (MEK) or in the presence of a water azeotroping solvent which is at least one selected from the group consisting of toluene, xylene, methylcyclohexane, methyl isobutyl ketone, hexane, cyclohexane and heptane.

The method can further comprise removing water from the liquid bottoms to increase concentration of MXA in the liquid bottoms.

Preferably, the MXA solid is substantially free, in particular completely free, of adipamic acid, adipamide and adipimide.

According to an eighth aspect, the invention encompasses a method of producing AA including providing a fermentation derived MgA-containing solution, adding at least one of AA, NH₃, NH₄⁺, and Mg⁺² to the broth to preferably maintain the pH of the broth below 6, distilling/evaporating the MgA-containing solution to form an overhead that comprises water and, optionally, ammonia, and a liquid bottoms that comprises MgA, crystallizing the MgA into a solid from the MgA-containing solution by cooling/evaporative/antisolvent crystallization, dissolving the MgA solid in water, converting the MgA to an AA-containing solution by at least one of electrodialysis, anion exchange using a cationic resin, and cation exchange using an anionic resin, and crystallizing by cooling/evaporative crystallization the AA from the AA containing solution generated during conversion of the MgA to AA.

In a preferred embodiment, distilling the broth and/or the MgA-containing solution is carried out in the presence of an ammonia separating solvent which is at least one selected from the group consisting of diglyme, triglyme, tetraglyme, sulfoxides, amides, sulfones, polyethyleneglycol (PEG), gamma butyrolactone (GBL), butoxytriglycol, N-methylpyrolidone (NMP), ethers, and methyl ethyl ketone (MEK) or in the presence of a water azeotroping solvent which is at least one selected from the group consisting of toluene, xylene, methylcyclohexane, methyl isobutyl ketone, hexane, cyclohexane and heptane.

The method can further comprise removing water from the liquid bottoms to increase concentration of MgA in the liquid bottoms.

Preferably, the MgA solid is substantially free, in particular completely free, of adipamic acid, adipamide and adipimide.

According to a ninth aspect, the invention relates to a method of producing fermentation derived DXS-containing solutions, where DXS comprises at least some DAS and, optionally, at least one of disodium succinate (DNaS) or dipotassium succinate (DKS), distilling/evaporating the DXS-containing solution to form an overhead that comprises water and ammonia and a liquid bottoms that comprises MXS, where MXS is at least one of monoammonium succinate (MAS), monosodium succinate (MNaS) or monopotassium succinate (MKS), and at least some DXS, crystallizing the MXS into a solid from the DXS-containing solution by cooling/evaporative/antisolvent crystallization, dissolving the MXS solid in water, converting the MXS to a SA containing solution by the addition of a strong acid, optionally concentrating the SA containing solution, and crystallizing by cooling/evaporative crystallization the SA from the SA containing solution.

Preferably, the strong acid is H₂SO₄ or HCl.

Finally, the invention relates to a method of producing fermentation derived DXA-containing solutions, where DXA comprises at least some DAA and, optionally, at least one of disodium adipate (DNaA) or dipotassium adipate (DKA), distilling/evaporating the DXA-containing solution to form an overhead that comprises water and ammonia and a liquid bottoms that comprises MXA, where MXA is at least one of monoammonium adipate (MAA), monosodium adipate (MNaA) or monopotassium adipate (MKA), and at least some DXA, crystallizing the MXA into a solid from the DXA-containing solution by cooling/evaporative/antisolvent crystallization, dissolving the MXA solid in water, converting the MXA to a AA containing solution by the addition of a strong acid, optionally concentrating the AA containing solution, and crystallizing by cooling/evaporative crystallization the AA from the AA containing solution.

Preferably, the strong acid is H₂SO₄ or HCl.

It will be appreciated that the following description is intended to refer to specific examples of steps in the methods according to the invention selected for illustration in the drawings and is not intended to define or limit the disclosure, other than in the appended claims.

Methods for conversion of MAS, MAA, MXS and/or MXA to SA or AA, respectively, include electrodialysis, anion exchange, and cation exchange, and combinations thereof. For ease of description much of the following disclosure will be directed to producing SA. However, the disclosure applies to AA as well as other dicarboxylic acids. Also, much of the disclosure will be oriented to MAS and DAS. The availability of MXS in solid form and its reconstitution in water enables providing a high quality feed to electrodialysis, anion exchange, and/or cation exchange for the conversion of MXS to SA. High quality of MXS feed means that it is highly reproducible, highly pure, and highly concentrated. The high quality enables attainment of substantially higher operational efficiency from electrodialysis, anion exchange, and/or cation exchange operations that can be used for the conversion of MXS to SA.

MAS or MXS can be converted to SA by the addition of strong mineral acids such as H₂SO₄, HCl, or the like. However, this method typically consumes a molar excess of the mineral acid and consequently generates a molar excess of the conjugate salt. Then SA can be crystallized from the salt solution. However, multiple crystallization steps may be required to obtain highly pure SA.

Accordingly, the invention provides methods for converting DAS and DXS to SA using MAS/MXS as a high quality intermediate. One challenge in developing integrated downstream purification technology for fermentation-derived products is the design considerations for process variability, particularly in batch-to-batch methods. Such variability is introduced into processes due to the use of cheap nutrient sources such as corn steep liquor (CSL). The composition of CSL varies from season to season, geography to geography, and production batch to batch leading to wide variations in constituents and compositions. This can be problematic in the efficient production of SA.

Furthermore, conventional downstream conversion technologies such as electrodialysis and ion exchange often have strict functional specifications wherein they demand high quality feed containing very small amounts of multivalent cations (< 5ppm) and coagulatable proteins (< 200 ppm). The effect of excess multivalent cations such as Ca⁺² and Mg⁺², particularly on electrodialysis, can be problematic. This can lead to premature replacement of expensive membranes. Protein coagulation can lead to process inefficiencies since the membranes and their respective compartments need to be subjected to clean-in-place protocols, thereby interrupting production.

Therefore, significant effort has been spent on designing upstream processes to substantially remove impurities prior to downstream processing. Such substantial removal helps meet functional specifications for downstream equipment as well as provide a better chance of producing products with acceptable quality. Typically, methods such as concentration and diafiltration using cross-flow filtration and deionization using ion exchange are used.

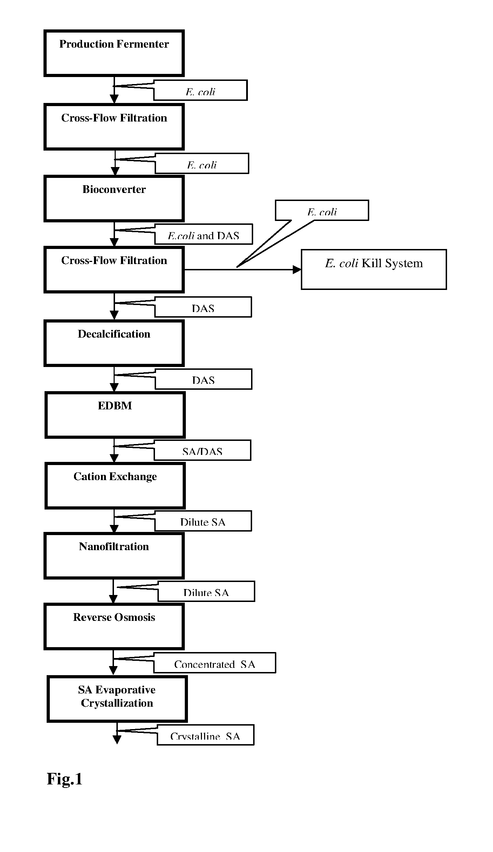

One improved process the invention operates utilizes such methods as shown in Fig. 1. The downstream purification technology implemented in that process may include two cross-flow filtration methods using microfiltration membranes, decalcification, electrodialysis, cation exchange, cross-flow filtration using nanofiltration membranes, and reverse osmosis to convert, isolate and purify SA. It was found that the process can be overwhelmed by the concentration of impurities and batch-to-batch variability of raw materials and fermentation/bioconversion. Although the process is functionally sound and operational, the process involves significant efforts to keep the equipment working efficiently. Examples include multiple regenerations of ion exchange columns, multiple clean-in-place cycles for membranes, multiple steam-in-place cycles for membranes and the like.

Furthermore, known fermentation/bioconversion generate numerous byproducts including other carboxylic acids such as acetic acid, formic acid, malic acid, fumaric acid and the like. Depending on the microorganism used in bioconversion, the byproduct acid concentration can be as high as 20 wt% of the total carboxylic acids. DXS, where DXS may be DNaS when a Na base is used, DKS when a K base is used, DMgS when a Mg base is used, or DAS when an ammonia (NH₄⁺ or NH₃) base is used. The DXS is converted to SA to derive SA from the fermentation broth.

However, DAS or DXS can be converted to the corresponding half-acid half-salt of SA using the processes disclosed in Published US Application No. 20110237831 the subject matter of which is incorporated herein by reference. US '831 teaches that the DXS can be converted to MXS to derive MXS from the fermentation broth, where MXS may be MNaS when a Na base is used, MKS when a K base is used, MMgS when a Mg base is used, or MAS when an ammonia (NH₄⁺ or NH₃) base is used. Subsequently, MXS can be crystallized and reconstituted in water to provide a high quality feed for conversion to SA using several methods. While crystallization of MXS is not necessary, it is preferred for further conversion to SA since the crystallization step improves the quality of the MXS solution. When electrodialysis and ion exchange methods are used to directly convert DAS/DXS-containing broths to SA, energy and material gets used to convert byproduct acids salts to their acid form. This is unavoidable and leads to excess consumption of energy and material contributing to unfavorable economics.

The inventor discovered that MAS/MXS, the half acid and half salt of SA, can be produced by thermally cracking DAS/DXS under relatively mild conditions and subsequently crystallized in high yield. These processes are disclosed in the aforementioned US '831.

The inventor found that the ability to crystallize MAS/MXS earlier in the overall process provides methods to isolate a substantially cleaner feedstock that can be used for downstream conversion and purification steps such as electrodialysis and ion exchange. In practice, MAS/MXS is reconstituted in water to provide a high quality feedstock for downstream conversion steps such as electrodialysis and ion exchange. The high quality MAS/MXS feedstock - highly reproducible, high concentration, high purity - enables highly efficient operation of downstream steps. In effect, MAS/MXS crystallization modulates transport of impurities and process disturbances introduced by upstream batch-to-batch variability in feedstocks and fermentation/bioconversion.

Further, MAS/MXS crystallization substantially prevents transport of byproduct acids (typically disposed as waste) through the downstream conversion steps such as electrodialysis and ion exchange. Therefore, energy and material are not spent to convert the salts of byproduct acids to their acid forms. Typically, these byproduct acids are disposed as waste. In our methods disclosed herein, where MAS/MXS is isolated in crystalline form as an intermediate, the byproduct acids are disposed of prior to expending energy and material for their conversion. This leads to substantial economic advantages.

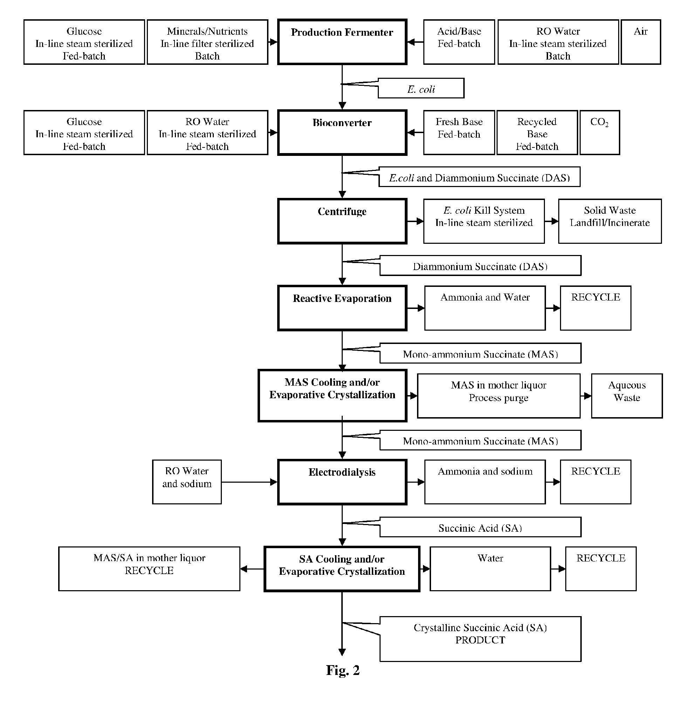

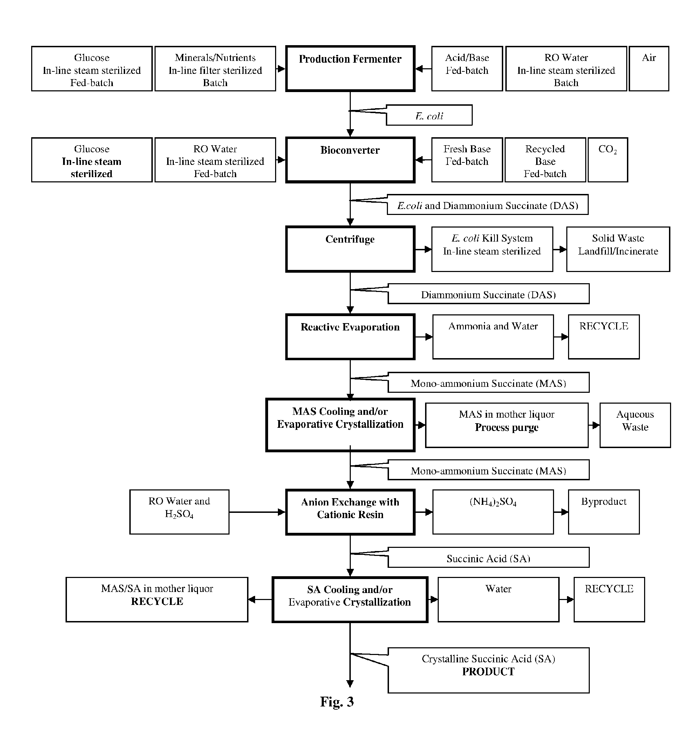

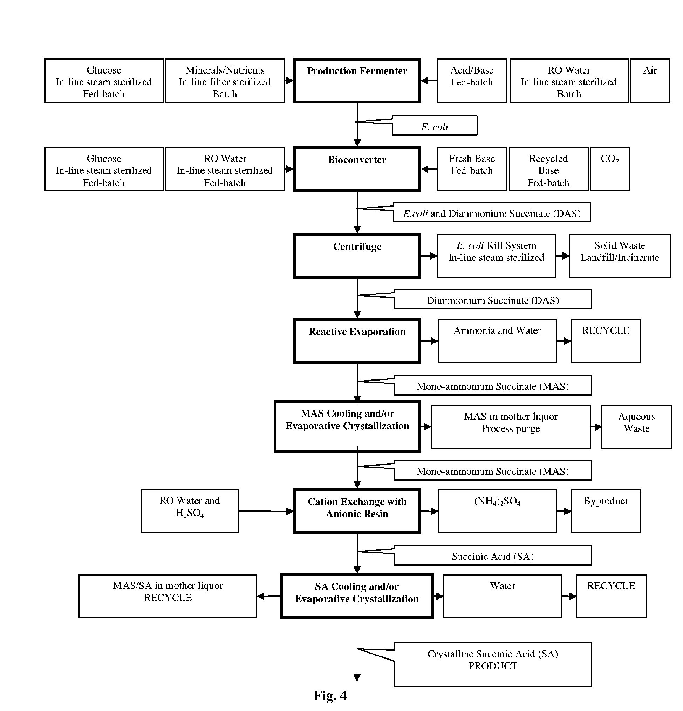

Examples of selected aspects of our process are described with reference to Figs. 2, 3 and 4.

### STEP 1: Production Fermenter

This step grows the *E. coli* production culture to convert glucose to SA. Prior to the production cycle, the vessel and connected process and utility pipes should be cleaned and steamed in place to minimize impact from microbial contamination.

The following raw materials may preferably be provided as one example:
Aqueous glucose, in-line steam sterilized, batch/fed-batch;
Aqueous minerals and nutrients, in-line filter sterilized, batch;
RO water, in-line steam sterilized, batch;
NH₃ and H₂S0₄, fed-batch;
Aeration, in-line filter sterilized.

The production culture may be grown aerobically to a concentration of about 10 - about 30 g/L over about 6 - about 11 hours and the growth rate may be controlled by fed-batch addition of glucose. The pH may be controlled at about 6.7 and the temperature may be controlled from about 37 to about 40°C. Subsequently, the culture may be directly transferred to a Bioconverter.

### STEP 2: Bioconverter

This step uses the *E*. *coli* culture to convert glucose to SA. Prior to the production cycle, the vessel and connected process and utility pipes may be cleaned in place to minimize impact from microbial contamination.

The following raw materials may preferably be provided as one example:
Aqueous glucose , in-line steam sterilized, fed-batch;
RO water, in-line steam sterilized, batch;
Fresh NH₃ , fed-batch;
Recycled NH₃ , fed-batch;
CO₂, in-line filter sterilized.

Bioconversion of glucose to SA using the culture may be conducted over about 20 - about 40 hours to produce about 40 - about 120 g/L of SA. The pH may be controlled at about 6.7 leading to the production of DAS. The temperature may be controlled at about 37°C. The pressure may be controlled at about 0 - about 3.7 psig. Subsequently, the unclarified DAS containing broth may be transferred to a set of centrifuges for clarification.

### STEP 3: Centrifuge

This step separates the DAS containing broth from the *E. coli* culture in preparation for downstream processing. The centrifuges may be cleaned in place prior to each batch per manufacturer recommendations.

Preferably, a set of three centrifuges (such as Alfa-Laval clarifiers) may be used with a design concentration factor of, for example, 40x. However, any number of centrifuges may be used, as appropriate. The concentrate may be about 20 wt% solids and can run through an in-line steam sterilizer to deactivate the culture. Subsequently, the deactivated culture may be transported to a landfill or transported for incineration. Other methods such as cross flow filtration may be used for clarifying broth.

The clarified DAS containing broth is transferred to the Reactive Evaporation step.

### STEP 4: Reactive Evaporation

This step converts DAS to MAS and concentrates the MAS containing broth for crystallization of MAS. DAS may be converted to MAS by partial thermal deammoniation (about 50%).

The reactive evaporation may be conducted at about 135°C and about 50 psig leading to rapid deammoniation and concentration. The bottom product is an aqueous solution of MAS (MAS containing broth) concentrated by about 2x, for example, and may be continuously transferred to the MAS Evaporative Crystallization step. The overhead product may have about 50 wt% each of the fed ammonia and water, which may be held in a tank for recycling.

### STEP 5: MAS Evaporative Crystallization

This step crystallizes MAS. This is a primary purification step.

Crystallization of MAS may be conducted in a three-stage continuous crystallization system where the stages are operated at about 0 - 60°C, about 0 - 40°C, and about 0 - 20°C, for example. Other numbers of stages may be used, as needed. Crystallization will provide about 85 - 95 wt% yield as MAS and the balance about 5 - 15 wt% of MAS contained in the crystallization mother liquor can be purged as an aqueous waste.

Crystalline MAS is recovered as about 85-95 wt% solids and transferred to Step 6.

### STEP 6; OPTION 1: Biopolar Membrane Electrodialysis - EDBM

This step converts MAS to SA.

Crystalline MAS may be dissolved in Reverse Osmosis water (RO Water) at about 10 - about 60 wt% concentration and is used as the feed for the EDBM step. The acid compartment of a EDBM unit operation can produce SA at about 10 - about 50 wt% in aqueous solution. The base compartment of the EDBM unit operation can produce ammonia at about 2 - about 10 wt%. The conversion, may be conducted at about 35 - about 100°C and around ambient pressure.

Typically, if the feed to EDBM is MAS, then a small amount of a highly conductive cation such as Na⁺ is added to the base compartment to provide conductivity. Typically, a molar ratio NH₄⁺:Na⁺ of about 9:1 is sufficient for efficient operation.

However, if the feed to EDBM is, for example, DNaS, then the conductivity of the base compartment can be provided by the sodium cation itself.

The about 10 - about 50 wt% SA solution may be transferred to the SA Evaporative Crystallization step or, optionally, to any other purification stages prior to evaporation and crystallization. These purification stages may include cation exchange to reduce mineral or metal cations, anion exchange to reduce mineral anions, activated carbon treatment to reduce color, cross flow filtration with nanofiltration membranes to reduce soluble oligomers, etc.

The about 2 - about 10 wt% ammonia solution may be transferred to an ammonia stripper to recover a concentrated ammonia condensate. The concentrated ammonia condensate may be mixed with the ammonia solution recovered from the Reactive Evaporation step to produce an ammonia solution for recycling to the Bioconverter.

The EDBM step can be conducted in three compartment configuration with three membrane types: cation, anion, and bipolar. The feed may be a salt and results in three products - depleted salty feed, free acid, and free base. This design was typically only economical for use with salts of strong acid and a strong base. This is because the conductivity of a weak acid or a weak base is too low for use in essentially pure form in one or both of the compartments.

We provide methods for selected organic acids economically using three compartment EDBM. The three-compartment EDBM can then provide both purification and acidification of the feed stream.

In one instance, economic implementation of three-compartment EDBM is enabled for salts of strong bases with weak acids. An example is DNaS. In another instance, implementation of three-compartment EDBM is enabled for salts of weak bases with weak acids. An example is DAS.

EDBM can also be in two compartment mode with just two membrane types: cation and bipolar. The feed may be a salt and results in two products - the first product is an acidified salty feed side rich in free organic acid and contains some residual salt, and the second product is free base, such as about 2 - 10% NaOH, for example.

EDBM can also be a two compartment mode with just two membrane types: Anion and bipolar. An example is the separation of ammonium chloride. The feed may be a salt and results in two products - the first product is basic salty feed side rich in NH₄OH and can contain some residual salt, and the second product is free acid such as about 5% HCL, for example.

In one instance, economics are improved for a two-compartment EDBM enabled for salts of strong bases with weak acids. An example is MNaS. In another instance, implementation of two-compartment EDBM with cation membranes is enabled for salts of weak bases with weak acids. An examples is MAS.

Further, electrodialysis can be a four compartment electrodialysis unit with alternating cation and anion membranes. There are two feed streams, for example, an aqueous MAS or MNaS containing stream and an aqueous free acid such as HCl, acetic acid or the like. Both feed materials are decomposed and there are two resultant products - free SA and the ammonium or sodium salt of the feed free acid, for example, ammonium acetate or ammonium chloride.

Operation of an electrodialysis stack balances the conductivity on the feed, acid and base cells to avoid local excess current flow and other operational problems. This is achieved by control during a batch run between concentration factors to control the amount of water in different streams. In a continuous mode, achieving this control is more difficult, but those skilled in the art can achieve such balance.

### STEP 6; OPTION 2: Anion Exchange using Cationic Resin

This step converts MAS to SA.

Typically anion exchange uses a resin bed and ion exchange beads such as IRA-93 strong base anion resin although others may be used. The anion exchanger may also be in a flat sheet membrane ion exchanger, or it may be an immiscible liquid amine exchanger.

The process fluid containing MAS contacts the anion resin that may be in a OH-hydroxide form or a HCO₃- bicarbonate form. For the case of bicarbonate ions exchange for the succinate ions, the effluent is ammonium bicarbonate. This effluent is then used for feed to the fermentation, where the CO₂ is used by the organism and the ammonia is used for pH control. For the case of hydroxyl ions, the effluent is ammonium hydroxide and can be used for pH control.

The resin is then loaded with succinate ions that are eluted. A strong acid such as H₂SO₄, HCl or the like may be used and the sulfate, chloride or the like becomes bound to the resin and the free SA displaced. The resulting SA solution may be transferred to the evaporation and crystallization stages or, optionally, to any other purification stages prior to evaporation and crystallization.

The resin is then loaded with sulfate, chloride ions or the like that are eluted. A base such as NH₄HCO₃, NH₄OH or the like is used for regeneration and ammonium sulfate, ammonium chloride or the like waste is generated and the resin returned to the bicarbonate (or hydroxide) form.

For each mole of MAS feed, about half a mole of H₂SO₄ and about one mole of NH₄HCO₃ (or NH₄OH) are preferably used. Alternatively, about one mole of HCL and about one mole of NH₄HCO₃ (or NH₄OH) are used. There is one product (SA aqueous solution) and two effluent streams (recyclable base solution and waste salt solution).

Anion exchange using cation resin can also be used to remove residual impurity anions such as PO4-. A base such as NH₄HCO₃, NH₄OH, NaOH or the like is used and phosphate salt waste is generated and the resin returned to the bicarbonate (or hydroxide) form. STEP 6; OPTION 3: Cation Exchange using Anionic Resin

This step converts MAS to SA.

A free acid is generated by treating the solution with a cation exchange process. Typically, cation exchange uses a resin bed and ion exchange beads such as Dowex50W-X8 or Amberlite IR120-H⁺ or the like, in the free acid form. A strong acid cation exchange resin is used inasmuch as a weak acid cation exchange resin has very little effective capacity below about pH 5.5. The strong acid cation exchanger may also be in a flat sheet membrane ion exchanger, or it may be an immiscible liquid strong acid cation exchanger.

The process fluid containing MAS contacts the cation resin that may be in a H-hydrogen form. The effluent is a solution of SA as NH₄⁺ carried with MAS is exchanged for the H⁺ on the resin. The SA solution may be transferred to the evaporation and crystallization stages or, optionally, to other purification stages.

The resin is then loaded with NH₄⁺ that is eluted. A strong acid such as H₂SO₄ or the like is used and the H⁺ from H₂SO₄ becomes bound to the resin and the NH₄⁺displaced producing a (NH₄)₂SO₄ solution. The resin is then regenerated and ready for the next production cycle. The (NH₄)₂SO₄ solution is disposed of.

For each mole of MAS feed, about 0.7 mole of H₂SO₄ is used and there is one product (SA aqueous solution) and one effluent streams (waste salt solution).

### STEP 7: SA Evaporative Crystallization

This step concentrates and crystallizes SA. This is a secondary purification step.

Evaporation and crystallization of SA may be conducted in multi-stage continuous evaporation and crystallization systems, for example, where the evaporation stages may be operated at about 80°C, and about 40°C, while the crystallization stages may be operated at about 40°C, and about 20°C. Other numbers of stages and operating conditions may be used as needed. The crystallization can provide about 90 - about 95 wt% yield as SA and the balance about 5 - about 10 wt% of SA contained in the crystallization mother liquor may be recycled to the reactive evaporation step.

Crystalline SA may be recovered as about 90 wt% solids and transferred to the solids handling operations including drying, sieving, and packaging.

### EXAMPLE 1

### USE OF PURE MAS - THREE COMPARTMENT EDBM

A three compartment EDBM system may include: (1) feed compartment (feed and retentate), (2) acid compartment, and (3) base compartment. A purified MAS solution is fed to the feed compartment to begin the process. Upon application of an appropriate voltage across the compartments, the feed is depleted of succinate anions and NH₄⁺. The resulting solution is collected as the retentate. In the acid compartment, the succinate anion combines with H⁺ to form SA and in the base compartment NH₄⁺ combines with water to form NH₃.

The following Table 1 shows the material balance and stream properties resulting from an Aspen-Plus program:

**Table 1**

| | FEED | PRODUCTS | | |
|---|---|---|---|---|
| | | RETENTATE | ACID | BASE |
| Succinate (g/L) | 118 | 113.1 | 163.94 | 0 |
| NH₄⁺ (wt%) | 1.78 | 1.57% | 0 | 0.08% |
| NH₃ (wt%) | 0 | 0 | 0 | 7.84% |
| Conductivity (mS/cm) | 75.1 | 47.8 | 3.69 | 5.62 |
| Temperature (C) | 60 | 60 | 60 | 60 |
| pH | 4.52 | 4.44 | 2.08 | 14.79 |

For three compartment EDBM, there is an additional consideration that the base compartment and the acid compartment have low conductivities. The low base side conductivities can be overcome by adding some sodium hydroxide to the base product. The base side product may be then recycled over a stripping column to recover ammonia. The acid concentrate, SA, also has low conductivity - this can be increased by adding some sodium succinate to the acid side. The sodium may be subsequently removed by an ion exchange finishing step. This three compartment mode of operation provides purification of the SA as well as acidification.

Optionally, as the feed side is depleted of MAS, additional MAS can be added to maintain the feed side conductivity. Additional MAS can be added to the feed side to hold the feed side concentration at about 10% to about 15% for the duration of the run. For 100 liters of feed side solution, successive batches of dry MAS of about 10 kg each can be added as long as impurities in the MAS do not affect the membranes.

### EXAMPLE 2

### USE OF PURE MAS - TWO COMPARTMENT EDBM - CATION + BIPOLAR

A two compartment EDBM system may include: (1) acid compartment, and (2) base compartment. A purified MAS solution is fed to the acid compartment to begin the process. Upon application of an appropriate voltage across the compartments, the feed is depleted of NH₄⁺. The resulting solution is rich in the acid form of SA and collected as the product stream. In the base compartment, NH₄⁺ combines with water to form NH₃.

The following Table 2 shows the material balance and stream properties resulting from an Aspen program:

**Table 2**

| | | PRODUCTS | |
|---|---|---|---|
| | FEED | BASE | ACID |
| Concentration Succinates g / L | 118.01 | - | 132.81 |
| Concentration NH₄⁺ (wt%) | 1.8% | 0.1% | 1.0% |
| Concentration NH₃ (wt%) | 0.0% | 7.8% | 0.0% |
| pH (ASPEN) | 4.49 | 14.5 | 3.98 |
| Conductivity, CALC, mS/cm | 77.61 | 7.22 | 54.94 |
| Temperature deg C | 40 | 40 | 40 |

For two compartment EDBM, there is an additional consideration that the base compartment has a low conductivity. The low base side conductivity can be overcome by adding some sodium hydroxide to base product. The base side product is then recycled over a stripping column to recover ammonia.

### USE OF A MXS CONTAINING SOLUTION - TWO COMPARTMENT EDBM-CATION + BIPOLAR

In this example, a EUR6B-40-bip two compartment pilot bipolar membrane electrodialysis stack provided by Ameridia of Summerset, NJ, USA was used.

A substantially purified MXS containing solution was fed to the acid compartment to begin the process. Upon application of an appropriate voltage across the compartments, the feed is depleted of NH₄⁺ and Na. The resulting solution is rich in the acid form of SA and collected as the product stream. The initial conductivity of the acid compartment was 65 mS/cm and the final conductivity was 5.5 mS/cm. In the base compartment, NH₄⁺ and Na combines with water to form NH₃ and Na containing base solution. The experiment was conducted at 50 °C.

The following Table 3 shows initial and final composition for the acid compartment solution.

**Table 3**

| **Measurement** | **Units** | **Acid Compartment Initial Feed** | **Acid Compartment Final Product** |
|---|---|---|---|
| **Total** | [kg] | 47.8 | 44.2 |
| **Succinic Acid** | [mg/mL] | 145.229 | 135.096 |
| **Acetic Acid** | [mg/mL] | <0.5 | <0.5 |
| **Na** | [ppm] | 1369 | 264 |
| **NH4** | [ppm] | 17219 | 655 |

The table shows that the ammonium and sodium levels decreased by about 96.5% and 80%, respectively, with a yield of ~86% succinic acid. The majority of the succinic acid losses were attributed to residual product within the ED stack piping and acid holding tank.

### EXAMPLES 4-5

Instead of substantially purified MAS as in Examples 1-2, a MAS that contains at least about 5% sodium succinate or potassium succinate are used. This reduces the need for adding sodium ion to control conductivity in various steps.

### EXAMPLES 6-7

Instead of substantially purified MAS as in Examples 1-2, MNaS, MKS, or mixtures thereof are used as the feed.

### EXAMPLE 8

### USE OF PURE MAS - ANION EXCHANGE

In this example, the strong base anion exchange resin is a solid bead. An aqueous MAS solution is pumped (21.02 kgmol/hour of MAS and 1077 kgmol/hour water), optionally through heat exchange to adjust the temperature to a condition suitable for good ion exchange kinetics and low bed pressure drop, to the ion exchange system.

The ion exchange system includes four resin beds, where at a given moment the beds are in the following operating modes, but not in any particular order:
- one resin bed being used for treating the feed stream (loading) and recovering the ammonium bicarbonate effluent (succinate displaces biocarbonate from the resin)
- one resin bed being used for unloading free SA using HCl and recovering SA in a aqueous solution (Cl⁻ displaces succinate from the resin)
- one resin bed being used for regeneration with NH₄HCO₃ to give bicarbonate form resin and ammonium chloride waste (bicarbonate displaces Cl- from the resin), and
- one resin bed in standby to allow smooth transition from treating to regeneration operations.

The system produces three stream: (1) the product SA stream (mainly SA and is approximately at a pH of 2.17 and is about 11% wt SA in solution at 60°C), (2) a NH₄HCO₃ stream for recycle to the fermentation section of the process, and (3) a waste solution comprising ammonium chloride.

The following Table 4 shows the material balance and stream properties resulting from an Aspen program:

**Table 4**

| | FEED | ACID | SALT RECYCLE | SALT WASTE |
|---|---|---|---|---|
| Mole Flow kmol/hr | | | | |
| WATER | 1,077 | 1,094 | 4,000 | 4,000.00 |
| SUCCINIC | 3.99 | 17.01 | - | - |
| HSUC⁻ | 13.04 | 3.96 | - | - |
| SUC⁻ | 3.99 | 0.05 | - | - |
| HCO₃⁻ | | - | 25.00 | - |
| H₃O⁺ | | 0.01 | 4.00 | 4.00 |
| NH₄⁺ | 21.06 | 4.06 | 21.00 | 21.00 |
| CL⁻ | 0 | 0 | - | 25 |
| Total Flow kg/hr | 22,249 | 22,266 | 74,041 | 73,402 |
| Temperature C | 60.00 | 60.00 | 60.00 | 60.00 |
| Pressure kPa | 250.00 | 101.33 | 101.33 | 101.33 |

Operation can not be performed at higher temperatures with standard anion resins, but newer resins may allow higher temperatures.

### EXAMPLE 9

### USE OF PURE MAS - CATION EXCHANGE

In this example, the strong acid cation exchange resin is a solid bead. An aqueous MAS solution is pumped (21.02 kgmol/hour of MAS and 1077 kgmol/hour water), optionally through heat exchange to adjust the temperature to a condition suitable for good ion exchange kinetics and low bed pressure drop, to the ion exchange system.

The ion exchange system includes three resin beds, where at a given moment the beds are in the following operating modes, but not in any particular order:
- one resin bed being used for treating the feed stream and produces an aqueous solution of SA (NH₄⁺ displaces H⁺ from the resin)
- one resin bed being used for regeneration using H₂SO₄ and recovering NH₄HSO₄ effluent as waste (H⁺ displaces NH₄⁺ from the resin), and
- one resin bed in standby to allow smooth transition from treating to regeneration operations.

The system produces two stream: (1) the product SA stream (mainly SA and is approximately at a pH of 2.17 and is about 11% wt SA in solution at 60°C), and (2) a waste solution comprising NH₄HSO₄.

The following Table 5 shows the material balance and stream properties resulting from an Aspen program:

**Table 5**

| | FEED Solution | PRODUCT Solution | SULFURIC ACID FEED | WASTE SALT SOLUTION |
|---|---|---|---|---|
| Mole Flow kmol/hr | | | | |
| WATER | 1,077 | 1,098 | 4,000 | 4,000 |
| NH₃ | 0.00 | 0.00 | - | 0.00 |
| SUCCINIC | 3.99 | 20.83 | - | - |
| HSUC⁻ | 13.04 | 0.19 | - | - |
| SUC⁻ | 3.99 | 0.00 | - | - |
| H₃O⁺ | 0.00 | 0.13 | 25.00 | 4.00 |
| NH₄⁺ | 21.06 | 0.06 | - | 21.00 |
| HSO₄⁻ | - | - | 25.00 | 25.00 |
| Temperature C | 60.00 | 60.00 | 60.00 | 60.00 |
| Pressure kPa | 250.00 | 101.33 | 101.33 | 101.33 |
| PH | 4.40 | 2.17 | 0.47 | 1.60 |

Operation can be performed at higher temperatures if desired, but lower temperatures will require operation at lower concentrations of succinic acid to avoid precipitation.

### EXAMPLE 10

### USE OF MXS CONTAINING SOLUTION- CATION EXCHANGE

A cation column was loaded with 5.9kg XA2023Na resin which was 16 equivalents. The MXS containing solution (Acid Compartment Final Product from Example 3) was passed through the column at 4BV/hr (bed volumes) using a peristaltic pump in a upflow mode. The solution was maintained at 50 °C during the experiments. After passing all the acid through the column, a 2.3L water wash was used to remove any residual acid from the column. Regeneration involved 19L 1N HCl followed by 15L water wash. Results can be seen in table 6.

**Table 6**

| **Measurement** | **Units** | **Into Strong Cation** | **Out of Strong Cation** |
|---|---|---|---|
| **Total** | [kg] | 44.2 | 40.1 |
| **Succinic Acid** | [mg/mL] | 135.096 | 128.134 |
| **Acetic Acid** | [mg/mL] | <0.5 | <0.5 |
| **Na** | [ppm] | 264 | 17.6 |
| **NH4** | [ppm] | 655 | 95.9 |

In this experiment, the mostly acidified acid compartment final product from Example 3 was used demonstrating that the mostly acidifies acid product can be substantially acidified using cation exchange. The example shows about a 94% reduction in sodium and about a 87% reduction in ammonium leading to further acidification by cation exchange. In principal, cation exchange can be used prior to partial acidification using electrodialysis to achieve the desired final acidification.

### EXAMPLES 11-14

Instead of substantially purified MAS as in Examples 1-5, a MAS that contains at least about 5% sodium succinate or potassium succinate are used. This reduces the need for adding sodium ion to control conductivity in various steps.

### EXAMPLES 15-18

Instead of substantially purified MAS as in Examples 1-5, MNaS, MKS, or mixtures thereof are used as the feed.

Although apparatus and methods have been described in connection with specific forms thereof, it will be appreciated that a wide variety of equivalents may be substituted for the specified elements and steps described herein without departing from the spirit and scope of this disclosure as described in the appended claims.

### EXAMPLE 19

### CONCENTRATION AND CRYSTALLIZATION OF SA

In this example, SA that has been substantially acidified using a combination of bipolar membrane electrodialysis (two compartment) and cation exchange with anion resin is concentrated and crystallized.

The evaporation set up consisted of a 60°C concentration vessel equipped with a hot water system to provide heat for evaporation and a vacuum system consisting of a cooled condenser (glycol/water at 4°C), an acetone/dry ice trap, and a vacuum pump. A condensate collector was also attached to the condenser.

An about 12 wt% substantially acidified SA solution was concentrated to an about 30 wt% SA solution. About 23 kg of water was removed from about 37.9 kg of the substantially acidified SA solution leaving about 14.9 kg of SA solution for Crystallization. The condensate water was tested for organic acids and results gave readings below the detection limits.

The concentrated SA solution was cooled to 4 °C for crystallization of SA by adding the solution in a stirred vessel placed in an environmental chamber. The crystals were harvested by filtration giving 5.1 kg wet crystals. About 1 kg of wet crystals were removed to be tested for a drying method while the remaining 4.1 kg was dried to ultimately give 3 kg dry crystals with a moisture content of 0.276 wt%. About 8.15kg of mother liquor was recovered while 1.30 kg of water wash was used. The data are presented in Table 7.

**Table 7**

| **Measurement** | **Units** | **Feed** | **Condensate** | **Supernatant** | **Water Wash** | **Crystals** |
|---|---|---|---|---|---|---|
| **Total** | [kg] | 37.9 | 23 | 8.15 | 1.3 | 3 |
| **Succinic Acid** | [mg/mL] | 120.952 | | 38.982 | 40.883 | |
| **Acetic Acid** | [ppm] | <500 | | 718.469 | 649.209 | <57 |
| **Melting Point** | [°C] | | | | | 186.3 |
| **Moisture** | [%] | | | | | 0.276 |

## Claims

1. A method of producing fermentation derived DXS-containing solutions, where DXS comprises at least some DAS and, optionally, at least one of disodium succinate (DNaS) or dipotassium succinate (DKS), distilling/evaporating the DXS-containing solution to form an overhead that comprises water and ammonia and a liquid bottoms that comprises MXS, where MXS is at least one of monoammonium succinate (MAS), monosodium succinate (MNaS) or monopotassium succinate (MKS), and at least some DXS, crystallizing the MXS into a solid from the DXS-containing solution by cooling/evaporative/antisolvent crystallization, dissolving the MXS solid in water, converting the MXS to a SA containing solution by at least one of electrodialysis, anion exchange using a cationic resin, and cation exchange using an anionic resin, and crystallizing by cooling/evaporative crystallization the SA from the SA containing solution generated during conversion of the MXS to SA.

2. The method of claim 1, wherein distilling the DXS-containing solution is carried out in the presence of an ammonia separating solvent which is at least one selected from the group consisting of diglyme, triglyme, tetraglyme, sulfoxides, amides, sulfones, polyethyleneglycol (PEG), gamma butyrolactone (GBL), butoxytriglycol, N-methylpyrolidone (NMP), ethers, and methyl ethyl ketone (MEK) or in the presence of a water azeotroping solvent which is at least one selected from the group consisting of toluene, xylene, methylcyclohexane, methyl isobutyl ketone, hexane, cyclohexane and heptane.

3. The method of claim 1 or 2, further comprising removing water from the liquid bottoms to increase concentration of MXS in the liquid bottoms.

4. The method of any of claims 1 to 3, wherein the MXS solid is substantially free of DXS, succinamic acid, succinamide and succinimide.

5. A method of producing SA including providing a fermentation derived DAS-containing solution comprising DAS and magnesium succinate (MgS), distilling/evaporating the DAS-containing solution to form an overhead that comprises water and ammonia, and a liquid bottoms that comprises MgS, crystallizing the MgS into a solid from the DAS-containing solution by cooling/evaporative/antisolvent crystallization, dissolving the MgS solid in water, converting the MgS to an SA-containing solution by at least one of anion exchange using a cationic resin, and cation exchange using an anionic resin, and crystallizing by cooling/evaporative crystallization the SA from the SA containing solution generated during conversion of the MgS to SA.

6. The method of claim 5, wherein distilling the DAS-containing solution is carried out in the presence of an ammonia separating solvent which is at least one selected from the group consisting of diglyme, triglyme, tetraglyme, sulfoxides, amides, sulfones, polyethyleneglycol (PEG), gamma butyrolactone (GBL), butoxytriglycol, N-methylpyrolidone (NMP), ethers, and methyl ethyl ketone (MEK) or in the presence of a water azeotroping solvent which is at least one selected from the group consisting of toluene, xylene, methylcyclohexane, methyl isobutyl ketone, hexane, cyclohexane and heptane.

7. The method of claim 5 or 6, further comprising removing water from the liquid bottoms to increase concentration of MgS in the liquid bottoms.

8. The method of any of claims 5 to 7, wherein the MgS is substantially free of DAS, succinamic acid, succinamide and succinimide.

9. A method of producing SA including providing a fermentation derived MXS-containing solution, where MXS comprises at least one of MAS, MNaS or MKS, optionally, adding at least one of SA, NH₃, NH₄⁺, Na⁺, and K⁺ to the broth to preferably maintain the pH of the broth below 6, distilling/evaporating the MXS-containing solution to form an overhead that comprises water and, optionally, ammonia, and a liquid bottoms that comprises MXS, crystallizing the MXS into a solid from the MXS-containing solution by cooling/evaporative/antisolvent crystallization, dissolving the MXS solid in water, converting the MXS to an SA-containing solution by at least one of electrodialysis, anion exchange using a cationic resin, and cation exchange using an anionic resin, and crystallizing by cooling/evaporative crystallization the SA from the SA containing solution generated during conversion of the MXS to SA.

10. The method of claim 9, wherein distilling the MXS-containing solution is carried out in the presence of an ammonia separating solvent which is at least one selected from the group consisting of diglyme, triglyme, tetraglyme, sulfoxides, amides, sulfones, polyethyleneglycol (PEG), gamma butyrolactone (GBL), butoxytriglycol, N-methylpyrolidone (NMP), ethers, and methyl ethyl ketone (MEK) or in the presence of a water azeotroping solvent which is at least one selected from the group consisting of toluene, xylene, methylcyclohexane, methyl isobutyl ketone, hexane, cyclohexane and heptane.

11. The method of claim 9 or 10, further comprising removing water from the liquid bottoms to increase concentration of MXS in the liquid bottoms.

12. The method of any of claims 9 to 11, wherein the solid MXS is substantially free of succinamic acid, succinamide and succinimide.

13. A method of producing SA including providing a fermentation derived MgS-containing solution, adding at least one of SA, NH₃, NH₄⁺, and Mg⁺² to the broth to preferably maintain the pH of the broth below 6, distilling/evaporating the MgS-containing solution to form an overhead that comprises water and, optionally, ammonia, and a liquid bottoms that comprises MgS, crystallizing the MgS into a solid from the MgS-containing solution by cooling/evaporative/antisolvent crystallization, dissolving the MgS solid in water, converting the MgS to an SA-containing solution by at least one of anion exchange using a cationic resin, and cation exchange using an anionic resin, and crystallizing by cooling/evaporative crystallization the SA from the SA containing solution generated during conversion of the MgS to SA.

14. The method of claim 13, wherein distilling the MgS-containing solution is carried out in the presence of an ammonia separating solvent which is at least one selected from the group consisting of diglyme, triglyme, tetraglyme, sulfoxides, amides, sulfones, polyethyleneglycol (PEG), gamma butyrolactone (GBL), butoxytriglycol, N-methylpyrolidone (NMP), ethers, and methyl ethyl ketone (MEK) or in the presence of a water azeotroping solvent which is at least one selected from the group consisting of toluene, xylene, methylcyclohexane, methyl isobutyl ketone, hexane, cyclohexane and heptane.

15. The method of claim 13 or 14, wherein the MgS solid is substantially free of succinamic acid, succinamide and succinimide.
